# EUROPEAN PATENT SPECIFICATION

(11) **EP 0 614 605 B1**
(45) Date of publication and mention of the grant of the patent: **16.02.2000**
(21) Application number: 94301764.0
(22) Date of filing: 11.03.1994
(51) Int. Cl.: A01K 29/00, A01K 67/02

(54) **Method to improve fattening in mammals**
Verfahren zur Steigerung der Gewichtszunahme von Säugetieren
Méthode pour l'amélioration de l'engraissage des mammifères

(30) Priority: 12.03.1993 AR 32449893
(43) Date of publication of application: 14.09.1994
(73) Proprietor: Turin, Enrique Horacio, (2700) Pergamino, Province of Buenos Aires (AR)
(72) Inventor: Turin, Enrique Horacio, (2700) Pergamino, Province of Buenos Aires (AR)
(74) Representative: Pendlebury, Anthony

(56) References cited:
- EP-A- 0 117 163
- FR-A- 2 290 906
- FR-A- 2 445 139
- GB-A- 2 079 158
- GB-A- 2 154 875

## Description

### BACKGROUND OF THE INVENTION

The present invention basically relates with a device which was designed to be inserted in the uterine cavity of mammals, particularly in cows and calfs, in order to improve fattening and handling of the animals. "Handling" should be interpreted in the scope of this invention as the action of placing the animals in different pasture parcels to assure an effective treatment.

Intents of weight gain were effected in the past based on animal castration. In 1906, Livio Dutto reported in "Cow castration" chirurgic practices of Nufer in this matter with ulterior ovary ectomy. Furthermore, in the past century, Charlier and Degive carried out bloody techniques of bonding ovaries. Dutto designed an ovarytome, an instrument which improved Degive's techniques and this instrument was still used at present. However, it acts in bloody and cruel manner and success is variable and essentially depends from the ovaries bond position and their peritoneal revascularization.

It is known that weight gain is a main object in cattle breeding, but another object of this invention is the use of an unbloody method to be applied to the animals. The present invention leads to this object by means of an unbloody technique which enables the production of anestrous (absence of oestral period). The sexual cycle in the animals consists of four phases: 1) pro-oestrum which is the period in which increases the activity of the generative organs; 2) oestrum or mating period which is the only time when the female is willing to mate and coition is fruitful 3) metoestrum wherein the activity of the organs is decreasing and 4) dioestrum which is the period of relative quiescence before the next proestrum.

Specific hormones are involved in the compliance of this cycle. The ovulation is produced during the oestrum which is the time when the mature ovule is released by the ovary. Once ovulation is produced, the *luteus corpus* is developed and produces Progesterone, an hormone of large anabolic effect responsive of maintaining pregnancy. In the case that fecundation is not produced the *luteus corpus* is desintegrated by the action of another hormone, Prostaglandine F2, and consequently a new sexual cycle starts. Prostaglandine is formed in the endometrium. Basically, if Prostaglandine F2 is not developed, *luteus corpus* would persist in forming progesterone causing two important effects: a) anabolic and 2) absence of oestrum.

The combination of both effects in significant increase in the weight gain of the animals.

One of the objects of this invention is a method of improving fattening in mammals. A further object of the invention is to enable male and female mammals to coexist in the same pasture parcel at the same time without the risk of a female mammal becoming pregnant.

Achievement of this object of the invention makes it possible to guarantee the selling of non-pregnant mammals

### DETAILED DESCRIPTION INCLUSIVE OF PREFERRED EMBODIMENTS

The device to be introduced in the uterine cavity of the mammal consists of a body of synthetic material having an inverted V-shape surrounded with a copper filament. In order to enable extraction from the uterine cavity the body can be provided in one of the extremes with a tail, preferably of synthetic material.

In a preferred embodiment of the invention the body is of a diameter of 1mm and a length of 3 to 7 mm. The synthetic material constituting the body is nylon and if it is provided the tail is also constituted by nylon with a diameter of 0.3 mm and a length of about 70 cm.

Basically, this device is located in the uterus of the mammal. The persistent irritation produced in the endometrium plus the action of the copper avoids the production of Prostaglandine F2, the animal lacks of oestrum and consequently the progesterone is released in view of the persistent *luteus corpus*.

The significant extra weight gain produced in part originates by the anabolic effect of progesterone but also by the absence of metabolic and energetic consumption in the oestrum period.

The method of applying the device consists in immobilizing the animal and introducing in its uterine cavity a pipette carrying the device at the end. The pipette locates the device in the endometrium and then the pipette is extracted from the animal. Prior to introduction into the animal, the inseminating pipette having the device at the end is submerged in a disinfectant solution basically constituted by 10% iodine or 10% lugol.

Assays were carried out with cows and calfs in order to demonstrate daily weight gain.

The animals were not treated with any hormone before effecting the assays., and they were separated in six groups indicated as A, B, C, D, E and F. Each of the groups was treated with paraticide. Results are reflected in Table 1.

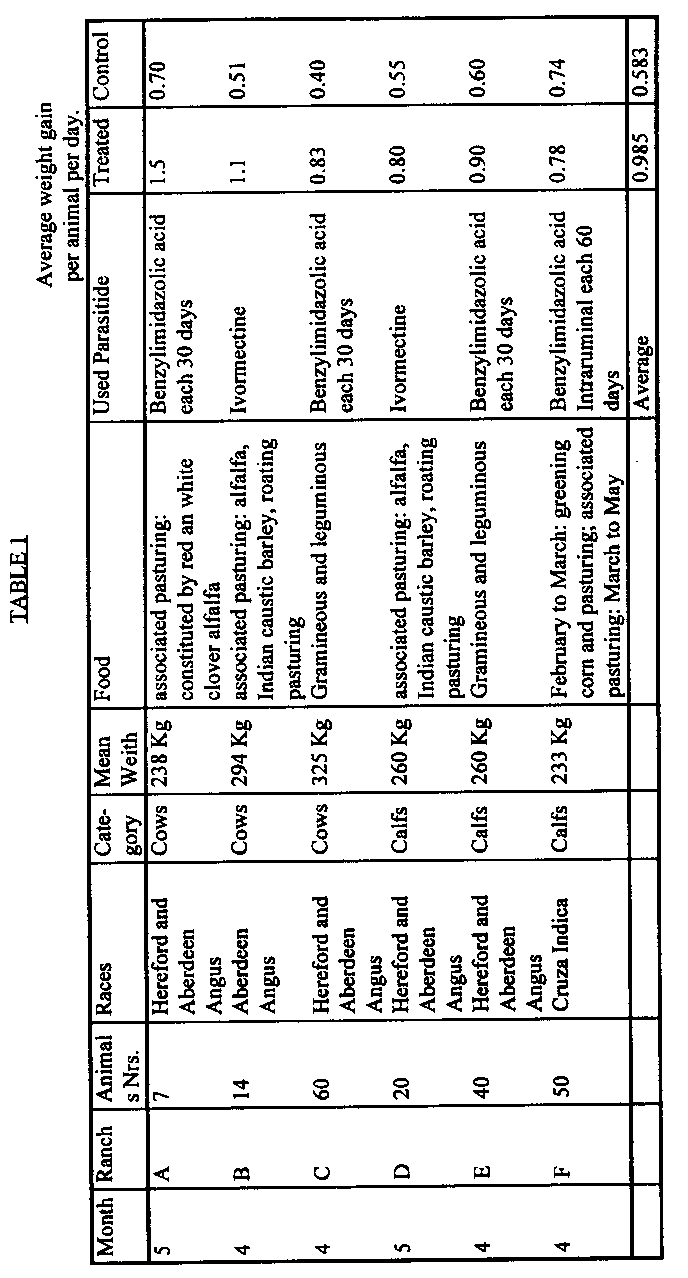

As reflected in Table 1 the treated animals show a significative weight dally gain as compared with control animals. The amounts of weight gain depend from specific factors such as race, category, food etc..

Assays were also performed to study progesterone level in animals treated in accordance with the invention. Results are given in Table 2.

**TABLE 2**

| Normal Values 0.25 - 1.5 ng/ml - Folicular phase Cows treated with the invention > 0.4 ng/ml - Luteum phase | | | | | | | |
|---|---|---|---|---|---|---|---|
| Sample | Progesterone Level (ng/ml) | Sample | Progesterone Level (ng/ml) | Sample | Progesterone Level (ng/ml) | | |
| 1 | (lesser than) 0.47 | 11 | (lesser than) 0.47 | 21 | 0.96 | | |
| 2 | 0.47 | 12 | 0.47 | 22 | 2.6 | | |
| 3 | 0.47 | 13 | 0.99 | 23 | 0.63 | | |
| 4 | 0.49 | 14 | 4.3 | 24 | 0.98 | | |
| 5 | 2.3 | 15 | 0.59 | 25 | 0.47 | | |
| 6 | 0.71 | 16 | 0.51 | 26 | 2.6 | | |
| 7 | (lesser than) 0.47 | 17 | 2.9 | 27 | 1.6 | | |
| 8 | 0.53 | 18 | 1.6 | 28 | 0.98 | | |
| 9 | 0.47 | 19 | 4.4 | 29 | 0.79 | | |
| 10 | 0.89 | 20 | 0.93 | 30 | 0.71 | | |

| Control Cows (untreated) | | | | | | | |
|---|---|---|---|---|---|---|---|
| Sample | Progesterone Level (ng/ml) | Sample | Progesterone Level (ng/ml) | Sample | Progesterone Level (ng/ml) | Sample | Progesterone Level (ng/ml) |
| 1 | 4.1 | 6 | 7.9 | 11 | 5.3 | 16 | 1.2 |
| 2 | 2.6 | 7 | 7.9 | 12 | 5.4 | 17 | 9.1 |
| 3 | 1.0 | 8 | 1.1 | 13 | 17 | 18 | 10.4 |
| 4 | 4.6 | 9 | 5.4 | 14 | 4.5 | 19 | 9.4 |
| 5 | 5.8 | 10 | 1.6 | 15 | 7.8 | | |

The progesterone levels were studied in the 30 treated animals ad the 19 control animals The treated animals maintained a continuous rage between 0,45 ad 1,5 ng/ml which reflected the absence of oestrum. On the other hand, the control cows showed discontinuous values, some about 7-11 ng/ml in the upper level and 1-3 in the lower level.

## Claims

1. Use of an intrauterine device to be introduced into the uterine cavity of a mammal for the purpose of improving fattening in the mammal by producing a persistent luteus corpus in the absence of oestrum in the mammal without hormone treatment.

2. Use according to claim 1 wherein the device comprises a body of a synthetic material formed in an inverted "V" shape which is surrounded with a copper filament.

3. Use according to claim 2 wherein the synthetic material is nylon.

4. Use according to claim 2 or claim 3 wherein one end of the body is provided with a tail for facilitating extraction of the device from the uterine cavity of the mammal.

## Patentansprüche

1. Verwendung einer intrauterinen Vorrichtung zur Einführung in den Uterushohlraum eines Säugers zum Zweck der Steigerung der Gewichtszunahme des Säugers durch Erzeugung eines dauerhaften Gelbkörpers (corpus luteum) in Abwesenheit der Brunft beim Säuger ohne Hormonbehandlung.

2. Verwendung gemäß Anspruch 1, bei dem die Vorrichtung einen Körper aus einem synthetischen Material umfaßt, das in Form eines inversen "V" ausgebildet und von einem Kupferfilament umgeben ist.

3. Verwendung gemäß Anspruch 2, wobei das synthetische Material Nylon ist.

4. Verwendung gemäß Anspruch 2 oder 3, wobei ein Ende des Körpers mit einem Schwanz zum Erleichtern des Herausziehens der Vorrichtung aus dem Uterushohlraum des Säugers versehen ist.

## Revendications

1. Utilisation d'un dispositif intra-utérin destiné à être introduit dans la cavité utérine d'un mammifère afin d'améliorer l'engraissement de ce mammifère en produisant un corps jaune persistant en l'absence d'oestrus chez ce mammifère sans traitement hormonal.

2. Utilisation suivant la revendication 1, dans laquelle le dispositif comprend un corps constitué d'une matière synthétique sous forme d'un "V" inversé qui est entouré par un filament en cuivre.

3. Utilisation suivant la revendication 2, dans laquelle la matière synthétique consiste en un Nylon.

4. Utilisation suivant la revendication 2 ou la revendication 3, dans laquelle une extrémité du corps est munie d'une queue pour faciliter l'extraction du dispositif de la cavité utérine du mammifère.
